# EUROPEAN PATENT SPECIFICATION

(11) **EP 3 595 693 B1**
(45) Date of publication and mention of the grant of the patent: **15.12.2021**
(21) Application number: 18767525.1
(22) Date of filing: 16.03.2018
(51) Int. Cl.: A61K 36/73, A61K 47/42, A61P 1/08, A61P 25/04, A61P 25/08

(54) **CANNABINOID FORMULATIONS AND DOSAGE**
CANNABINOID-FORMULIERUNGEN UND DOSIERUNG
FORMULATIONS ET DOSAGE DE CANNABINOÏDES

(30) Priority: 16.03.2017 US 201762472329 P
(43) Date of publication of application: 22.01.2020
(73) Proprietor: Izun Pharmaceuticals Corp., New York, NY 10020 (US)
(72) Inventor: COHEN, Shmuel, 99833 Nehosha (IL); LEVINE, William Z., 93232 Jerusalem (IL)
(74) Representative: Schulz Junghans Patentanwälte PartGmbB
(86) International application number: PCT/IL2018/050307
(87) International publication number: WO 2018/167795

(56) References cited:
- WO-A1-2015/140736
- GB-A- 2 384 707
- US-B1- 6 383 513
- PAPA V M ET AL: "The effects of pH and temperature on the in vitro bindings of delta-9-tetrahydrocannabinol and other cannabinoids to bovine serum albumin", JOURNAL OF PHARMACEUTICAL AND BIOCHEMICAL ANALYSIS, ELSEVIER B.V, AMSTERDAM, NL, vol. 8, no. 4, 1 January 1990 (1990-01-01) , pages 353-356, XP026525949, ISSN: 0731-7085, DOI: 10.1016/0731-7085(90)80049-U [retrieved on 1990-01-01]
- SHARMA, P. et al.: "Global Need for Novel Herbal Drug Formulations", Int J Phcog Phytochem Res, vol. 8, no. 9, 10 September 2016 (2016-09-10), pages 1535-1544, XP055549619,

## Description

### BACKGROUND

Cannabis is a genus of plants comprising the species Cannabis sativa, C. indica, and C. ruderalis. Cannabis plants have been cultivated for a variety of uses including making fibers (hemp), medicinal use and recreational drug use. Cannabis is also commonly known as marijuana.

One of the most common ways that cannabis is used for medicinal use in many countries (also known as medical marijuana) is through smoking. Smoking cannabis is typically performed by using a pipe, by using a water-pipe (also known as a bong) which filters the smoke through water before inhalation or by rolling in paper to form marijuana cigarettes, also known colloquially as "joints." The part of the plant typically used for smoking is the whole flower and budding leaf.

Cannabinoids are compounds active on cannabinoid receptors in humans. Cannabinoids of plant origin, also known as phyto-cannabinoids, are abundant in plants of the Cannabis genus. Two known cannabinoids which are present in relatively high concentrations in Cannabis sativa are tetrahydracannabinol-acid (THCA) or its decarboxylated product tetrahydracannabinol (THC) and cannabidiolic acid (CBDA) or its decarboxylated product cannabidiol (CBD). Psychoactive and other medical effects of many of the cannabinoids have been studied. For example, THC was found to have psychoactive (calming) effects, analgesic effects, antioxidant effects and to increase appetite. CBD was found to have neuroprotective effects and to have ameliorative effects in patients with schizophrenia and Parkinson's disease.

Cannabinoid-containing compositions comprising at least one cannabinoid and at least one plasma protein, and methods for manufacture of such compositions have been disclosed in PCT application publication WO 2015/140736.

### SUMMARY

It has been found that compositions comprising cannabinoids bound to a plasma protein, upon administration to mammals, prolongs the therapeutic effect of the cannabinoids in the mammal, when compared to administration of the cannabinoids alone, in an unbound state. Accordingly, embodiments of the invention provide the claimed protein-bound cannabinoids for use in methods for treatment of disease claimed comprising administering to a patient in need thereof, the claimed amount of a composition comprising cannabinoids bound to plasma protein, in which the daily dosage administered is a reduced amount relative to the daily dosage of the cannabinoids not bound to plasma protein, in order to achieve the same therapeutic effect.

Additional embodiments provide the claimed protein-bound cannabinoids for use in for treatment of disease claimed comprising administering to a patient in need thereof, the claimed amount of a composition comprising cannabinoids bound to plasma protein in which the composition is administered less frequently than the administration of the cannabinoid alone, without being bound the plasma protein prior to administration, in order to achieve the same or better therapeutic effect as a same or higher dose of unbound cannabinoid.

The composition comprising cannabinoids in a plasma protein-bound form are administered at intervals of two-fold to three-fold duration compared to a same dose of cannabinoids in non-protein-bound form, such that, by way of example, where non-plasma protein-bound cannabinoid is typically treated with twice or thrice daily administration for a given condition, a once-daily administration of the same amount of cannabinoid in plasma protein-bound form provides a same or better therapeutic effect. As a result of the increased time interval between administrations, the total amount of cannabinoid administered in accordance with an embodiment of the disclosure, in the form of being bound to a protein, over a time period comprising multiple administrations is reduced to between one-half and one-third compared to unbound cannabinoid, while achieving a same or better therapeutic effect. Alternatively or in combination, the amount of cannabinoid per administration for the cannabinoid in protein-bound form is reduced, optionally to account for the longer duration of effectiveness or improved potency. Optionally, the amount of cannabinoid administered in the protein-bound form at each administration for achieving a same or better therapeutic effect is between 0.7-fold and 0.5-fold compared to the amount of unbound cannabinoid.

In an embodiment of the disclosure, for administration of a protein-bound cannabinoid, a prolonged, up to threefold, time interval for administration (compared to unbound cannabinoid) is combined with a reduced amount, up to half, of cannabinoid per administration (compared to unbound cannabinoid). A result of such a combination, when administered in the form of a protein-bound cannabinoid in accordance with an embodiment of the disclosure, the total amount of cannabinoid administered over a time period comprising multiple administrations is between about one-third and about one-sixth of the total amount of unbound cannabinoid administered over the same multiple-dose time period for achieving a same or better therapeutic effect.

In an embodiment of the disclosure, the plasma protein is non-covalently bound to the at least one cannabinoid, resulting in the formation of a protein-cannabinoid complex. Optionally, the non-covalent binding is characterized by non-specific lipophilic and polar interactions, by way of example with hydrophobic protein pockets in the plasma protein.

According to an embodiment of the invention, the composition comprises one of or a combination of more than one of albumin, lipoprotein, glycoprotein, α, β, and γ globulin and diluted blood plasma.

According to an embodiment of the invention, the composition comprises a cannabinoid selected from the group consisting of Δ9-THC, CBD, According to an embodiment of the invention, the composition comprises a mixture of cannabinoids. According to an embodiment of the invention, the cannabinoid is a synthetic cannabinoid. The synthetic cannabinoid is nabilone. According to the embodiment of the invention, the cannabinoid is THC. According to an embodiment of the invention, the plasma protein is an albumin. The albumin may comprise human serum albumin, bovine serum albumin or egg albumin (OVA).

This summary is provided to introduce a selection of concepts in a simplified form that are further described below in the detailed description. This summary is not intended to identify key features or essential features of the claimed subject matter, nor is it intended to be used to limit the scope of the claimed subject matter.

### BRIEF DESCRIPTION OF FIGURES

Non-limiting examples of embodiments are described below with reference to figures attached hereto that are listed following this paragraph. Identical structures, elements or parts that appear in more than one figure are generally labeled with a same numeral in all the figures in which they appear, and a numeral labeling an icon representing a given feature in a figure may be used to reference the given feature. Dimensions of components and features shown in the figures are chosen for convenience and clarity of presentation and are not necessarily shown to scale.

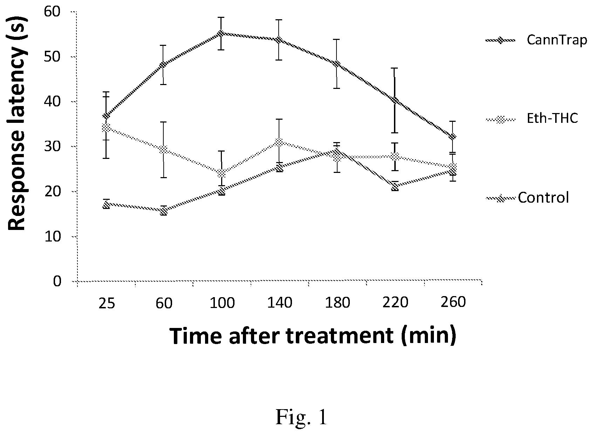

Fig. 1 depicts a line graph showing response latency of mice (in seconds) tested using a hot plate test at various time points administered one of: an ethanolic composition comprising cannabinoids without plasma protein (Eth-THC), a composition comprising cannabinoids bound to plasma protein (CannTrap), or a negative control (Control) comprising ethanol without cannabinoid;

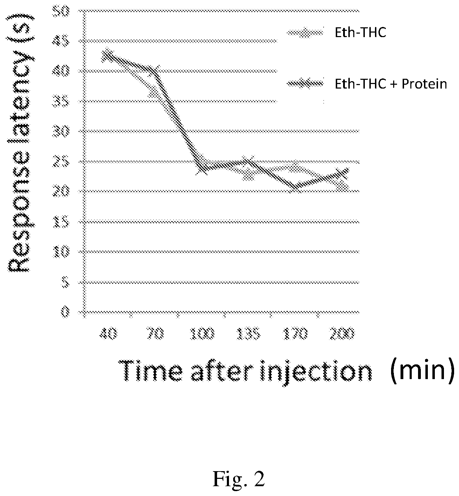

Fig. 2 depicts a line graph showing response latency of mice (in seconds) tested using a hot plate test at various time points administered one of: an ethanolic composition comprising cannabinoids (Eth-THC) without plasma protein and a mixed composition (Eth-THC + protein) comprising the Eth-THC composition mixed with a plasma protein solution 5 minutes prior to administration; and

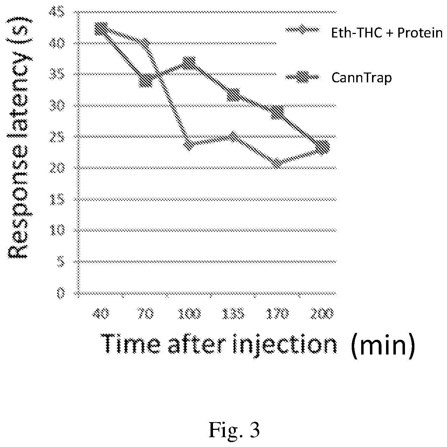

Fig. 3 depicts a graph response latency of mice (in seconds) tested using a hot plate test at various time points administrated one of: a solution comprising plasma protein and unbound cannabinoids (Eth-THC + protein), a composition comprising cannabinoids bound to plasma protein (CannTrap).

### DETAILED DESCRIPTION

### Example 1a: Preparation of an ethanolic solution comprising cannabinoids

Smoke of cannabis plant material high in THC was formed by burning, and the smoke was streamed through anhydrous ethanol. The solution was concentrated by evaporation of ethanol up to a total cannabinoid concentration of between 1.5 milligram (mg) per milliliter (ml) and 5 mg/ml, the concentration being determined using quantitative HPLC (high-pressure liquid chromatography) -based analysis.

### Example 1b: Preparation of "CannTrap", a composition comprising cannabinoids bound to plasma protein

100 microliters (µl) of the concentrated ethanolic solution prepared in accordance with Example 1a above was slowly added to 500µl of a 2 mg/ml solution of rice recombinant human serum albumin (HSA), a plasma protein. The cannabinoid-protein solutions were formed using ethanol and water in a 1:5 ratio. The cannabinoid-protein solutions were incubated under ventilated conditions at 30°C for 16 hours to allow slow ethanol evaporation from the solution to favor binding of cannabinoids with the HSA. After incubation, the cannabinoid-protein solutions were loaded onto a 0.5 ml Amicon 0.3 kilodalton (kDa) filter (Millipore). HSA molecules, which exceed the 0.3 kDa cutoff of the filter, as well as any compounds bound to the HSAs, would be unable to pass the filter, while compounds smaller than the 0.3 kDa cutoff, including free cannabinoids and fatty acids not bound to HSAs, as well as any residual ethanol, would pass through the filter. The complex was washed twice with 500µl of water. Protein, which included HSA bound with cannabinoids, was eluted from the amicon filter, resulting in a purified composition that is enriched in HSA-bound cannabinoid. This resulting composition is referred to herein as a "CannTrap" composition.

Cannabinoids and HSA concentrations in the CannTrap composition were determined using HPLC-based quantification and a bicinchoninic acid assay (BCA), respectively. The final concentration of the cannabinoids in complex with protein in the CannTrap composition depends on the final elution volume and can be up to 70 mg/ml or higher for HSA and up to 3 mg/ml or higher for cannabinoids, and the relative amount of cannabinoid to HSA can be between 20 micrograms (µg) and 100 µg of cannabinoid to 1 mg of HSA.

The relative quantity of various cannabinoids in each of the Eth-THC solution and the CannTrap composition, expressed as a percentage of total cannabinoid composition, is listed in Table 1 below:

**Table 1:**

| Cannabinoid | Eth-THC solution (% total cannabinoid) | CannTrap composition (% total cannabinoid) |
|---|---|---|
| CBDA | 0.5 | 1.2 |
| CBGA | 0.4 | 0.4 |
| CBG | 3.5 | 8.6 |
| CBD | - | 0.0 |
| CBN | 6.9 | 5.3 |
| THCA | 2.7 | 1.7 |
| THC | 83.0 | 80.9 |
| CBC | 3.0 | 1.8 |

### Example 2a: Testing analgesic effect of CannTrap composition

The hot plate pain test is a well-established murine model to test the analgesic effect of tested compounds. A head-to-head comparison of analgesic effect as assayed by the hot plate pain test was made between compositions comprising cannabinoids extracted from cannabis smoke in ethanol without plasma protein (the Eth-THC solution produced in accordance with Example 1a) with a composition comprising HSA-bound cannabinoinds (the CannTrap composition produced in according with Example 1b). Male C57BL/6JOlaHsd mice, aged 9-10 weeks at study initiation, were divided into 3 groups with 6-7 mice per group.

The results of the comparison are shown in Fig. 1. A first group was treated with the Eth-THC solution (indicated in Fig. 1 as "Eth-THC", having square plot markers), a second group was treated with the CannTrap composition comprising HSA-bound cannabinoid (indicated in Fig. 1 as "CannTrap", having diamond plot markers), and a third, control, group was administered ethanol only, without any cannabinoid (indicated in the figure as "Control", having triangular plot markers). The target THC dosage per injection was 5 mg THC / kilograms (kg) body weight.

The injected mice were then tested by placing each mouse on an electronically controlled hot plate (hotplate analgesia meter, Columbus Instruments, Columbus, Ohio, USA) heated to 51°C at 25, 60, 100, 140, 180, 220 and 260 minutes after injection, to test response latency in seconds. Latency was defined as time between the animal being placed on the hot plate surface and the animal showing a first sign of discomfort (licking its paw, shaking or jumping off to avoid thermal pain.) In attempt to reduce mistake of the identification of the first sign of discomfort, the first sign of discomfort of the front leg was disregarded, and times for first sign of hind-leg discomfort and first hind-leg licking were recorded. Results are shown as the mean time of the first sign of hind-leg discomfort and hind-leg licking.

The results show that treatment with 5 mg/kg THC in the form of an ethanolic cannabinoid solution (Eth-THC) provided analgesic effect, demonstrated in the form of longer response latency for the hot-plate test compared to response latency for control animals treated with ethanol vehicle was about 20 seconds. Treatment with the Eth-THC solution resulted in response latency increasing to a maximum of over 30 seconds (at 25 minutes post-injection), representing a 1.5-fold increase over control. The response latency decreased after 25 minutes and returned to baseline levels by 100 minutes post-treatment, thus showing that injection of ethanolic cannabinoid provided analgesic effect for a duration of less than 100 minutes.

By contrast, a 5 mg/kg dose of THC in the form of HSA-bound cannabinoid (CannTrap) provided significantly stronger analgesic effect for a longer duration. Treatment with the CannTrap composition resulted in response latency of about 35 seconds at 25 minutes post-injection, equivalent to ethanolic cannabinoid (Eth-THC). However, the response latency in the group treated the CannTrap composition continued to increase thereafter, increasing to a maximum of over 50 seconds at 100 minutes post-injection, representing a 2.5-fold increase in response latency over control. Therefore, HSA-bound cannabinoid was found to be about 1.7-fold (2.5 divided by 1.5) more potent than ethanolic cannabinoid for inducing analgesia. As such, administering the CannTrap composition at a reduced dose to administer about 3 mg/kg THC (0.58-fold dose compared to 5 mg/kg THC) would be expected to result in a similar maximum response latency as administering 5 mg/kg of unbound THC.

Moreover, the duration of the analgesic effect of HSA-bound cannabinoid treatment was longer compared to ethanolic cannabinoid, with the response latency approaching baseline at 260 minutes post-injection. Therefore, compared to ethanolic cannabinoid, which had already returned to baseline levels by 100 minutes post-injection, HSA-bound cannabinoid was found to induce analgesia for a duration of at least 2.6-fold longer than ethanolic cannabinoid.

### Example 2b: Analgesic effect of ethanolic cannabinoid solution compared to a mixture of HSA and cannabinoid not bound to the HSA

Reference is made to Figs. 2 and 3. Using the same hot plate assay, the analgesic effect of a solution comprising HSA and cannabinoid, in which the cannabinoids had not had optimal conditions to bind to HSA, was tested. The unbound mix solution (Eth-THC + Protein) was prepared by combining the ethanolic cannabinoid (Eth-THC) prepared in accordance with Example 1a with an aqueous solution comprising HSA immediately prior to intraperitoneal injection. The solution was prepared so that the final ethanol concentration of the unbound mixture solution was less than 5% and the THC concentration was 0.5 mg THC / ml. The unbound mix solution was injected within 5 minutes of preparation. Given the relatively low ethanol concentration of the Eth-THC + Protein solution and the shortness of time between preparation and administration, the presence of HSA-bound cannabinoid was expected to be negligible. The analgesic effect of injecting the unbound mix solution (Eth-THC + Protein) was compared to the analgesic effects of ethanolic cannabinoid (Eth-THC) and HSA-bound cannabinoid (CannTrap). Each mouse receiving a THC-comprising composition, (Eth-THC, Eth-THC + Protein, or CannTrap) had the composition administered at a dose of 5 mg THC / kg body weight.

A comparison of the analgesic effect the Eth-THC solution and the Eth-THC + Protein solution is shown in Fig. 2. It is found that the analgesic effect of the unbound mix solution (Eth-THC + Protein, shown in Fig. 2 with x-shaped plot markers) is substantially the same as the ethanolic cannabinoid solution (Eth-THC), shown in Fig. 2 with triangular plot markers), confirming that merely the presence of HSA in a same solution as cannabinoids does not confer the advantageous properties of HSA-bound cannabinoids. The time course of the analgesic effect of unbound mix solution was essentially the same as that of ethanolic cannabinoids, and the analgesic effect had dissipated by 100 minutes post-injection in both treatments. By contrast, as shown in Fig. 3, HSA-bound cannabinoid (CannTrap, shown with square plot markers) maintained analgesic effect until 200 minutes post-injection for, twice as long as the unbound mix solution (Eth-THC + Protein, shown with diamond plot markers).

These results indicate that a prolonged effect of the same dosage of cannabinoid may be achieved when cannabinoids are administered already bound to a plasma protein. The potential advantages are many and include the following advantages: 1. Lower cannabinoid dosages may be administered, in compositions comprising plasma protein-bound cannabinoids, to patients in need thereof to provide similar effects as higher cannabinoid dosage using non-plasma-protein-bound composition. 2. Side effects associated with administering cannabinoids may be decreased by using compositions comprising cannabinoids bound to plasma protein. 3. Accumulation of cannabinoid in tissue of patients may be lowered by using a lower daily dosage of plasma protein-bound cannabinoid 4. Frequency of administration may be altered to provide fewer administrations of compositions comprising plasma protein-bound cannabinoids to a patient in need thereof per day (or per other period of time) than required when administering cannabinoids without plasma proteins.

### Example 3: Clinical uses of composition comprising cannabinoid and plasma protein

CannTrap compositions comprising cannabinoid bound to protein (such as HSA) are be used in a clinical setting using alternate dosing schedules relative to those used for cannabinoids administered without plasma proteins. In accordance with an embodiment of the disclosure, compositions comprising cannabinoids in a plasma protein-bound (CannTrap) form are administered less frequently, by way of example with a dose interval that is two-fold to three-fold longer compared to same dose of cannabinoids in non-protein-bound form. As such, where non-plasma protein-bound cannabinoid is recommended for twice or thrice daily administration, a same dose of plasma protein-bound cannabinoid provides a same or better effectiveness when administered once a day. Alternatively or in combination, the amount of cannabinoid per administration may be reduced, optionally to account for the longer duration of effectiveness and/or improved potency of the protein-bound CannTrap cannabinoid. Table 2 and further examples below details exemplary methods of treatment according to dosing schedules that may be followed using CannTrap compositions comprising plasma protein-bound cannabinoid.

**Table 2:**

| Indication | Cannabinoid used | Known dosage of cannabinoid | Exemplary cannabinoid dosage for composition comprising plasma protein-bound cannabinoid |
|---|---|---|---|
| appetite stimulation | Δ-9-THC (dronabinol) | 5 mg/day (twice daily) | 1-5 mg/day once daily |
| appetite stimulation | Δ-9-THC (dronabinol) | 2.5 mg/day (once daily) | 0.2-1.25 mg/day once daily |
| emesis (nausea and vomiting) | Δ-9-THC (dronabinol) | 2.5mg - 40 mg/day (four times daily) | 2.5 - 40 mg/day twice daily |
| emesis | nabilone | 1 mg - 2 mg/day (twice daily) | 1-2 mg/day once daily |
| pain/ fibromyalgia | nabilone | 1mg - 2 mg/day (twice daily); 0.5 mg to 1 mg before sleep for improved sleep | Up to 1 mg/day once daily |
| chronic upper motor neuron syndrome/ central pain | nabilone | 1 mg/day (once daily) | 0.5 mg/day once daily |
| Epilepsy | CBD | 200 mg/day | 100 mg/day |
| Dravet syndrome | CBD | 20 mg/kg/day | 10 mg/kg/day |
| Lennox-Gastaut Syndrome | CBD | 20 mg/kg/day | 10 mg/kg/day |
| pain (multiple sclerosis related) | combination of CBD and THC (nabiximols) | 100 µl/spray comprising 27mg/ml THC (2.7 mg THC per spray) and 25 mg/ml CBD (2.5 mg CBD per spray), median of 5 sprays per day, up to 12 sprays per day. | 100 µl/spray comprising 27mg/ml THC and 25 mg/ml CBD, median of 2.5 sprays per day, up to 6 sprays per day. |

Given its improved potency and/or duration of action, a CannTrap protein-bound cannabinoid in accordance with an embodiment of the disclosure is expected to have a same or better therapeutic effect for stimulating appetite compared to unbound cannabinoid when administered with a total administered amount of cannabinoid over multiple doses that is between about one-sixth and one-half of the total administered amount of the same cannabinoid in unbound form. As such, the following further examples are also provided.

### Example 3A - Protein-bound THC for appetite stimulation

A typical dosing schedule for stimulating appetite, in subjects suffering from reduced appetite associated with, by way of example, chemotherapy or AIDS, with orally administered dronabinol (unbound Δ-9-THC) can be as low as 2.5 mg/day, which is equivalent to a weekly dose of about 17.5 mg/week. By way of example, a CannTrap HSA-bound Δ-9-THC in accordance with an embodiment of the disclosure, administered orally at a total dosage of between about 3 mg/week Δ-9-THC and about 9 mg/week Δ-9-THC, is as effective in stimulating appetite as 17.5 mg/week of dronabinol (unbound Δ-9-THC).

### Example 3B - Protein-bound THC for treating nausea

A typical dosing schedule for treating nausea (for example associated with chemotherapy) with orally administered Δ-9-THC can be as low as 2.5 mg/day four times a day, which is equivalent to a weekly dose of about 70 mg/week. By way of example, a CannTrap HSA-bound Δ-9-THC administered orally at a total dosage of between about 12 mg/week Δ-9-THC and about 35 mg/week Δ-9-THC is as effective in treating nausea as 70 mg/week of unbound Δ-9-THC.

### Example 3C - Protein-bound nabilone for treating nausea

A typical dosing schedule for treating nausea (for example associated with chemotherapy) with orally administered nabilone can be as low as 1 mg/day twice a day, which is equivalent to a weekly dose of 14 mg/week. By way of example, a CannTrap HSA-bound nabilone administered orally at a total dosage of between about 2 mg/week nabilone and about 7 mg/week nabilone is as effective in treating nausea as 14 mg/week of unbound nabilone.

### Example 3D - Protein-bound nabilone for treating pain associated with fibromyalgia

A typical dosing schedule for treating chronic pain associated with fibromyalgia with orally administered nabilone can be as low as 1 mg/day twice a day, which is equivalent to a weekly dose of 14 mg/week. By way of example, a CannTrap HSA-bound nabilone administered orally at a total dosage of between about 2 mg/week nabilone and about 7 mg/week nabilone is as effective in treating fibromyalgia-associated pain as 14 mg/week of unbound nabilone.

### Example 3E - Protein-bound nabilone for treating chronic pain

Another typical dosing schedule for treating chronic pain (by way of example associated with fibromyalgia, upper motor neuron syndrome and/or central pain syndrome) with orally administered nabilone can be as low as 0.5 mg before bedtime, which is equivalent to a weekly dose of 3.5 mg/week. By way of example, a CannTrap HSA-bound nabilone administered orally at a total dosage of between about 0.6 mg/week nabilone and about 1.75 mg/week nabilone is as effective in treating fibromyalgia-associated pain as 3.5 mg/week of unbound nabilone.

### Example 3F - Protein-bound THC for treating chronic pain

Another typical dosing schedule for treating chronic pain (by way of example associated with cancer) with orally administered THC can be as low as 5 mg daily, which is equivalent to a weekly dose of 35 mg/week. By way of example, a CannTrap HSA-bound THC administered orally at a total dosage of between about 6 mg/week THC and about 17.5 mg/week THC is as effective in treating chronic pain as 35 mg/week of unbound THC.

### Example 3F - Protein-bound CBD for treating epilepsy

A typical dosing schedule for treating epilepsy with orally administered CBD can be about 200 mg daily, which is equivalent to a weekly dose of 1400 mg/week. By way of example, a CannTrap HSA-bound CBD administered orally at a total dosage of between about 233 mg/week CBD and about 700 mg/week CBD is as effective in treating epilepsy as 1400 mg/week of unbound CBD.

### Example 3G - Protein-bound CBD for treating epilepsy

A typical dosing schedule for treating epilepsy with orally administered CBD can be as low as 5 mg/kg daily, which is equivalent to a weekly dose of 35 mg/kg/week. By way of example, a CannTrap HSA-bound CBD administered orally at a total dosage of between about 5.5 mg/kg/week CBD and about 17.5 mg/kg/week CBD is as effective in treating epilepsy as 35 mg/kg/week of unbound CBD.

### Example 3H - Protein-bound CBD for treating epilepsy

A typical dosing schedule for treating Dravet Sydrome (also known as severe myoclonic epilepsy of infancy) or epilepsy in patients of Lennox-Gastaut Syndrome with orally administered CBD can be about 20 mg/kg daily, which is equivalent to a weekly dose of 140 mg/kg/week. By way of example, a CannTrap HSA-bound CBD administered orally at a total dosage of between about 23 mg/week CBD and about 70 mg/week CBD is as effective in treating epilepsy as 350 mg/week of unbound CBD.

### Example 31 - Protein-bound CBD and THC for treating epilepsy

A typical dosing schedule for chronic pain (by way of example associated with nerve lesion, rheumatoid arthritis, cancer, and multiple sclerosis) with a combination of THC and CBD via oromucosal spray be as low as about 2.7 mg THC and about 2.5 mg CBD daily, which is equivalent to a weekly dose of about 19 mg/week THC with about 17.5 mg/week CBD. By way of example, a CannTrap HSA-bound CBD administered orally at a total dosage of between about 3.2 mg/week THC with about 2.9 mg/week CBD and about 9.5 mg/week THC with about 8.7 mg/week CBD is as effective in treating epilepsy as about 19 mg/week THC with about 17.5 mg/week CBD.

### Example 3J - Protein-bound CBD and THC for treating chronic pain

A typical dosing schedule for chronic pain (by way of example associated with nerve lesion, rheumatoid arthritis, cancer, and multiple sclerosis) with a combination of THC and CBD via oromucosal spray be as low as about 2.7 mg THC and about 2.5 mg CBD daily, which is equivalent to a weekly dose of about 19 mg/week THC with about 17.5 mg/week CBD. By way of example, a CannTrap HSA-bound CBD administered orally at a total dosage of between about 3.2 mg/week THC with about 2.9 mg/week CBD and about 9.5 mg/week THC with about 8.7 mg/week CBD is as effective in treating epilepsy as about 19 mg/week THC with about 17.5 mg/week CBD.

According to an embodiment of the invention, appetite stimulation may be treated in patients suffering from AIDS, including AIDS related weight loss. According to an embodiment of the invention, emesis may be treated in a patient receiving chemotherapy and suffering from chemotherapy induced emesis. According to an embodiment of the invention, pain may be treated in patients suffering from neuropathic pain, chronic pain, cancer, multiple sclerosis or post-operative pain.

The pharmaceutical compositions according to an embodiment of the invention are conveniently presented in unit dosage form and are prepared by any of the methods well known in the art of pharmacy. In an embodiment of the invention, the unit dosage form is in the form of a tablet, capsule, lozenge, wafer, patch, ampoule, vial, metered-dose inhaler (oral or nasal route) or pre-filled syringe.

In an embodiment of the disclosure, the at least one protein-bound cannabinoid of the present disclosure is administered in the form of a pharmaceutical composition comprising at least one protein-bound cannabinoid as an active component together with a pharmaceutically acceptable carrier or diluent. The pharmaceutical composition is optionally administered through oral administration, parenteral administration, or inhalation.

According to an embodiment of the invention, more than 90% of the cannabinoids in the composition is bound to plasma protein. According to an embodiment of the invention, the composition is substantially free from cannabinoid not bound to the plasma protein.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tableting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the components of this invention can be combined with various sweetening agents, flavoring agents, coloring agents, emulsifying agents and/or suspending agents, as well as such diluents as water, ethanol, propylene glycol, glycerin and various like combinations thereof. In an embodiment of the disclosure, plasma-protein-bound cannabinoid may be administered orally together with a protease inhibitor and/or a buffering agent. For intranasal administration, a pharmaceutical composition can take the form of solutions, suspensions, powders and the like.

The compositions according to embodiments of this invention may also be administered in a controlled release formulation such as a slow release or a fast release formulation. Such controlled release dosage composition may be prepared using methods well known to those skilled in the art.

For purposes of parenteral administration, aqueous solutions may be prepared. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes.

Pharmaceutical compositions according to embodiments of the invention may contain an active amount of 0.1%-95% of the protein-bound cannabinoid(s) (based on total cannabinoid weight) preferably 1%-70%.

In an embodiment of the invention, the daily dosage of the protein-bound cannabinoid, is between 0.1 µg and 1500 mg. Some embodiments of the invention relate to treatments as monotherapy, in which a protein-bound cannabinoid is a sole active pharmaceutical agent used to treat a disease. Some embodiments of the invention relate to combination therapies in which a protein-bound cannabinoid is used in combination with another active pharmaceutical agent to treat a disease. "In combination with" refers to both drugs being substantially effective in the body at a same time. Both drugs can be administered substantially at the same time, or both drugs can be administered at different times but have effect on the body at the same time.

There is therefore provided in accordance with the disclosure at least one protein-bound cannabinoid bound to a plasma protein for use as a medicament for treatment of a condition, wherein a total cannabinoid dose of the at least one protein-bound cannabinoid, administered over a time period comprising multiple administrations, is lower relative to a total cannabinoid dose of the at least one cannabinoid when administered in unbound form for achieving a same degree of therapeutic effect for treating the condition. There is also provided in accordance with the disclosure a method for treating a subject having or suffering from a condition and in need thereof, the method comprising administering to the subject an therapeutically effective amount of a composition comprising at least one cannabinoid bound to a plasma protein or portion thereof, in which a total cannabinoid dose of the at least one cannabinoid, administered over a time period comprising multiple administrations, is lower relative to a total cannabinoid dose of the at least one cannabinoid in unbound form for achieving a same degree of therapeutic effect.

In an embodiment of the disclosure, time intervals between administrations of the at least one plasma protein-bound cannabinoid is between about two-fold and about three-fold longer in duration compared to a dosing schedule for achieving the same degree of therapeutic effect with administration of the at least one cannabinoid in unbound form.

In an embodiment of the disclosure, the amount of cannabinoid administered at each of the multiple administrations of the at least one plasma protein-bound cannabinoid is between about 0.7-fold and about 0.5-fold lower compared of the amount of the at least one cannabinoid administered in unbound form.

In an embodiment of the disclosure, the total amount of the at least one protein-bound cannabinoid administered over the multiple administrations is between one-sixth and one-half relative to the total administered dose of the at least one cannabinoid in unbound form for achieving the same degree of therapeutic effect.

In an embodiment of the disclosure, the at least one protein-bound cannabinoid is one or the claimed combination of two of the group consisting of: Δ9-THC, CBD, THC , and nabilone.

In an embodiment of the disclosure, the plasma protein or portion thereof is selected from the group consisting of: an albumin, a lipoprotein, a glycoprotein, and α, β, and γ globulins, and mixtures of one or more thereof.

Optionally, the condition is reduced appetite, the at least one cannabinoid comprises Δ-9THC, and the at least one cannabinoid is administered at a dose of between 3 mg/week Δ-9-THC and 9 mg/week Δ-9 THC.

Optionally, the condition is nausea, the at least one cannabinoid comprises Δ-9THC, and the at least one cannabinoid is administered at a dose of between 12 mg/week Δ-9-THC and 35 mg/week Δ-9 THC.

Optionally, the condition is pain associated with fibromyalgia, the at least one cannabinoid comprises nabilone, and the at least one cannabinoid is administered at a dose of between 2 mg/week nabilone and 7 mg/week nabilone.

Optionally, the condition is chronic pain, the at least one cannabinoid comprises nabilone, and the at least one cannabinoid is administered at a dose of between 0.6 mg/week nabilone and 1.75 mg/week nabilone. Optionally, the chronic pain is associated with one or more of fibromyalgia, upper motor neuron syndrome, and central pain syndrome.

Optionally, the condition is chronic pain associated with cancer, the at least one cannabinoid comprises THC, and the at least one cannabinoid is administered at a dose of between 6 mg/week THC and 17.5 mg/week THC.

Optionally, the condition is epilepsy, the at least one cannabinoid comprises CBD, and the at least one cannabinoid is administered at a dose of between 233 mg/week CBD and 700 mg/week CBD.

Optionally, the condition is epilepsy, the at least one cannabinoid comprises CBD, and the at least one cannabinoid is administered at a dose of between 5.5 mg/kg/week CBD and 17.5 mg/kg/week CBD.

Optionally, the condition is epilepsy associated with Dravet Syndrome or Lennox-Gastaut Syndrome, the at least one cannabinoid comprises CBD, and the at least one cannabinoid is administered at a dose of between 23 mg/kg/week CBD and 70 mg/kg/week CBD.

Optionally, the condition is epilepsy, the at least one cannabinoid comprises CBD and THC, and the at least one cannabinoid is administered at a dose of between 3.2 mg/week THC with 2.9 mg/week CBD and 9.5 mg/week THC with 8.7 mg/week CBD.

Optionally, the condition is chronic pain, the at least one cannabinoid comprises CBD and THC, and the at least one cannabinoid is administered at a dose of between 3.2 mg/week THC with 2.9 mg/week CBD and 9.5 mg/week THC with 8.7 mg/week CBD. The chronic pain optionally is associated with one or more of a nerve lesion, rheumatoid arthritis, a cancer, and multiple sclerosis.

In the description and claims of the present application, each of the verbs, "comprise," "include" and "have," and conjugates thereof, are used to indicate that the object or objects of the verb are not necessarily a complete listing of components, elements or parts of the subject or subjects of the verb.

Descriptions of embodiments of the invention in the present application are provided by way of example and are not intended to limit the scope of the invention. The described embodiments comprise different features, not all of which are required in all embodiments of the invention. Some embodiments utilize only some of the features or possible combinations of the features. Variations of embodiments of the invention that are described, and embodiments of the invention comprising different combinations of features noted in the described embodiments, will occur to persons of the art. The scope of the invention is limited only by the claims.

## Claims

1. At least one protein-bound cannabinoid bound to a plasma protein for use as a medicament for treatment of a condition, wherein a total cannabinoid dose of the at least one protein-bound cannabinoid, administered over a time period comprising multiple administrations, is lower relative to a total cannabinoid dose of the at least one cannabinoid when administered in unbound form for achieving a same degree of therapeutic effect for treating the condition, and wherein the at least one protein-bound cannabinoid and dose administered are:
a. between 0.6 mg/week nabilone and 1.75 mg/week nabilone when the condition is chronic pain;
b. between 12 mg/week Δ-9-THC and 35 mg/week Δ-9 THC when the condition is nausea;
c. between 2 mg/week nabilone and 7 mg/week nabilone when the condition is pain associated with fibromyalgia
d. between 3 mg/week A-9-THC and 9 mg/week Δ-9 THC when the condition is reduced appetite;
e. between 6 mg/week THC and 17.5 mg/week THC when the condition is chronic pain associated with cancer;
f. between 233 mg/week CBD and 700 mg/week CBD when the condition is epilepsy;
g. between 5.5 mg/kg/week CBD and 17.5 mg/kg/week CBD when the condition is epilepsy;
h. between 23 mg/kg/week CBD and 70 mg/kg/week CBD when the condition is epilepsy associated with Dravet Syndrome or Lennox Gastaut Syndrome; or
i. between 3.2 mg/week THC with 2.9 mg/week CBD and 9.5 mg/week THC with 8.7 mg/week CBD when the condition is epilepsy or chronic pain.

2. The at least one protein-bound cannabinoid for use according to claim 1 wherein the chronic pain is associated with one or more of a nerve lesion, rheumatoid arthritis, a cancer, and multiple sclerosis.

## Patentansprüche

1. Mindestens ein proteingebundenes Cannabinoid, gebunden an ein Plasmaprotein, zur Verwendung als Medikament bei der Behandlung einer Erkrankung, wobei eine Gesamt-Cannabinoiddosis des mindestens einen proteingebundenen Cannabinoids, verabreicht über einen Zeitraum, umfassend mehrere Verabreichungen, niedriger ist relativ zu einer Gesamt-Cannabinoiddosis des mindestens einen Cannabinoids, wenn in ungebundener Form verabreicht, um einen gleichen Grad an therapeutischer Wirkung bei der Behandlung der Erkrankung zu erzielen, und wobei das mindestens eine proteingebundene Cannabinoid und die verabreichte Dosis sind:
a. zwischen 0,6 mg/Woche Nabilon und 1,75 mg/Woche Nabilon, wenn die Erkrankung chronische Schmerzen ist;
b. zwischen 12 mg/Woche Δ-9-THC und 35 mg/Woche Δ-9 THC, wenn die Erkrankung Übelkeit ist;
c. zwischen 2 mg/Woche Nabilon und 7 mg/Woche Nabilon, wenn die Erkrankung Schmerzen im Zusammenhang mit Fibromyalgie ist;
d. zwischen 3 mg/Woche Δ-9-THC und 9 mg/Woche Δ-9 THC, wenn die Erkrankung Appetitlosigkeit ist;
e. zwischen 6 mg/Woche THC und 17,5 mg/Woche THC, wenn die Erkrankung chronische Schmerzen im Zusammenhang mit Krebs ist;
f. zwischen 233 mg/Woche CBD und 700 mg/Woche CBD, wenn die Erkrankung Epilepsie ist;
g. zwischen 5,5 mg/kg/Woche CBD und 17,5 mg/kg/Woche CBD, wenn die Erkrankung Epilepsie ist;
h. zwischen 23 mg/kg/Woche CBD und 70 mg/kg/Woche CBD, wenn die Erkrankung Epilepsie im Zusammenhang mit Dravet-Syndrom oder Lennox-Gastaut-Syndrom ist; oder
i. zwischen 3,2 mg/Woche THC mit 2,9 mg/Woche CBD und 9,5 mg/Woche THC mit 8,7 mg/Woche CBD, wenn die Erkrankung Epilepsie oder chronische Schmerzen ist.

2. Das mindestens eine proteingebundene Cannabinoid zur Verwendung gemäß Anspruch 1, wobei der chronische Schmerz mit einer oder mehreren Nervenläsionen, rheumatoider Arthritis, einem Krebs und multipler Sklerose zusammenhängt.

## Revendications

1. Au moins un cannabinoïde fixé à une protéine, fixé à une protéine plasmatique, destiné à être utilisé en tant que médicament pour le traitement d'une affection, dans lequel une dose de cannabinoïde totale de l'au moins un cannabinoïde fixé à une protéine, administrée sur une période de temps comprenant de multiples administrations, est inférieure à une dose de cannabinoïde totale de l'au moins un cannabinoïde lors de l'administration sous une forme non fixée pour obtenir un même degré d'effet thérapeutique pour traiter l'affection, et dans lequel l'au moins un cannabinoïde fixé à une protéine et la dose administrée sont compris :
a. entre 0,6 mg/semaine de nabilone et 1,75 mg/semaine de nabilone lorsque l'affection est la douleur chronique ;
b. entre 12 mg/semaine de Δ-9-THC et 35 mg/semaine de Δ-9 THC lorsque l'affection est la nausée ;
c. entre 2 mg/semaine de nabilone et 7 mg/semaine de nabilone lorsque l'affection est de la douleur associée à une fibromyalgie ;
d. entre 3 mg/semaine de Δ-9-THC et 9 mg/semaine de Δ-9 THC lorsque l'affection est un appétit réduit ;
e. entre 6 mg/semaine de THC et 17,5 mg/semaine de THC lorsque l'affection est de la douleur chronique associée à un cancer ;
f. entre 233 mg/semaine de CBD et 700 mg/semaine de CBD lorsque l'affection est l'épilepsie ;
g. entre 5,5 mg/kg/semaine de CBD et 17,5 mg/kg/semaine de CBD lorsque l'affection est l'épilepsie ;
h. entre 23 mg/kg/semaine de CBD et 70 mg/kg/semaine de CBD lorsque l'affection est l'épilepsie associée à un syndrome de Dravet ou syndrome de Lennox-Gastaut ; ou
i. entre 3,2 mg/semaine de THC avec 2,9 mg/semaine de CBD et 9,5 mg/semaine de THC avec 8,7 mg/semaine de CBD lorsque l'affection est l'épilepsie ou la douleur chronique.

2. L'au moins un cannabinoïde fixé à une protéine selon la revendication 1, dans lequel la douleur chronique est associée à un ou plusieurs éléments parmi une lésion nerveuse, la polyarthrite rhumatoïde, un cancer et la sclérose en plaques.
